(19) Europäisches Patentamt
European Patent Office
Office européen des brevets

(11) EP 1 757 307 A1

(12) **EUROPEAN PATENT APPLICATION**
published in accordance with Art. 158(3) EPC

(43) Date of publication:
**28.02.2007 Bulletin 2007/09**

(21) Application number: **05751273.3**

(22) Date of filing: **10.06.2005**

(51) Int Cl.:
***A61K 45/00*** *(2006.01)* ***A61P 17/14*** *(2006.01)*
***C12N 5/10*** *(2006.01)* ***C12N 15/09*** *(2006.01)*

(86) International application number:
**PCT/JP2005/011094**

(87) International publication number:
**WO 2005/120575 (22.12.2005 Gazette 2005/51)**

(84) Designated Contracting States:
**DE FR GB IT**

(30) Priority: **14.06.2004 JP 2004175444**
**14.06.2004 JP 2004175407**

(71) Applicant: **Shiseido Company, Limited**
**Chuo-ku**
**Tokyo 104-8010 (JP)**

(72) Inventors:
- **EHAMA, R. c/o SHISEIDO RES. CENTER (SHIN-YOKOHAMA)**
**Kanagawa 2248558 (JP)**
- **KISHIMOTO, J. c/o SHISEIDO RES. C.(SHIN-YOKOHAMA)**
**Kanagawa 2248558 (JP)**
- **IDETA, R. c/o SHISEIDO RES. CENTER (SHIN-YOKOHAMA)**
**Kanagawa 2248558 (JP)**
- **SOMA, T. c/o SHISEIDO RES. CENTER (SHIN-YOKOHAMA)**
**Kanagawa 2248558 (JP)**

(74) Representative: **Santarelli**
**14, avenue de la Grande Armée**
**Boîte Postale 237**
**75822 Paris Cedex 17 (FR)**

(54) **METHOD OF REGENERATING FOLLICLES BY INHIBITING GENE CAPABLE OF INHIBTING FOLLICLE FORMATION OR ACTIVATING GENE CAPABLE OF INDUCING FOLLICLE FORMATION**

(57) A method is provided for regenerating hair follicles by suppressing the expression of one or a plurality of genes having the ability to inhibit hair follicle formation selected from the group consisting of S100a6, Ctgf, Gsto1, Gas6, Klf2, Thbs1 and Thbd, or up-regulating the expression of one or a plurality of genes having the ability to induce hair follicle formation selected from the group consisting of Tgfbi, Gas1, Thbs2, Ifi202A, Bmp7, Efna1, Efna3, Cidea, Serping1, MS1, Irf6, Fmod and Fxyd4.

Fig.1

(a) (b)

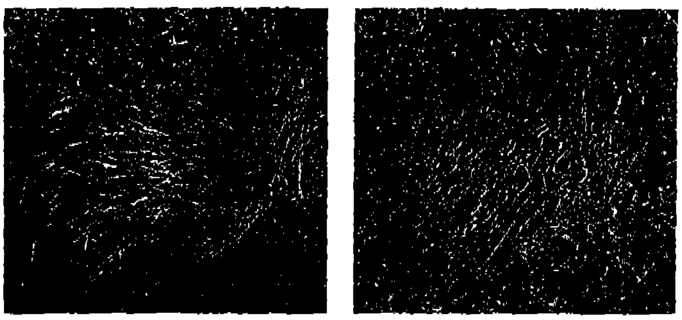

EP 1 757 307 A1

**Description**

## TECHNICAL FIELD

The present invention relates to a method for regenerating and/or forming hair follicles by attempting to suppress expression of a gene having the ability to inhibit hair follicle formation, and a method for culturing hair follicle dermal papilla cells by suppressing expression of that gene. Moreover, the present invention relates to a method for regenerating and/or forming hair follicles by attempting to accelerate expression of a gene having the ability to induce hair follicle formation, and a method for culturing hair follicle dermal papilla cells by up-regulating expression of that gene.

## BACKGROUND ART

There is a high demand for attempting to compensate for hair loss accompanying aging and so forth, and typically not only the use of hair tonics, but also hair transplants and other procedures are performed at health care institutions and so forth. On the other hand, there are considerable expectations, albeit somewhat excessive, being placed on regenerative medical technologies in the form of advanced medicine as an alternative to conventional organ transplants accompanying technical breakthroughs attributable to recent progress in stem cell research, a serious shortage of donors due to problems with tissue compatibility and so forth, and requirements based on ethical perspectives such as the issue of assessing brain death. Thus, attention unlike that in the past is being focused on hair follicle regenerative research as a model organ of regenerative medicine.

Research on the follicle formation mechanism in the developmental stage has been conducted comparatively extensively, and hair follicles have been determined to be formed as a result of a complex interaction attributable to signal transmission between epithelial cells (epidermal cells) and mesenchymal cells (hair follicle dermal papilla cells or DPC) lying directly beneath (R. Pause, at al., N. Engl. J. Med., 341, 491-497, 1999; K.S. Stenn, et al., Physiol. Rev. 81, 449-494, 2001; S.E. Miller, et al., J. Invest. Dermatol., 118, 216-225, 2002). In addition, once hair follicles have been formed, they undergo a cyclic regeneration in which anagen, catogen and telogen are repeated. Although numerous growth factors, cytokines, hormones, neuropeptides and other physiologically active substances are known to be involved in the regulation thereof, these physiologically active substances do not necessarily coincide with those involved in the mechanism of the developmental stage of hair follicle formation.

Based on the results of mouse hair follicle reconstitution experiments using nude mice, it has been determined that both epithelial cells and mesenchymal cells are essential for hair follicle regeneration, and that hair follicle regeneration is not induced unless a fixed number of cells are present (J. Kishimoto, et al., Proc. Natl. Acad. Sci., 96, 7336-7341, 1999). Moreover, although the regeneration of chimeric hair follicles comprised of mouse DPC and human epithelial cells has been suggested to be possible (Japanese Patent Application No. 2004-048332; Ehama, et al., 26th Annual Meeting of the Molecular Biology Society of Japan, Abstracts, 2PC-024, 2003), it is still not possible to regenerate completely human hair follicles. One of the reasons for this is that it is difficult to obtain an adequate amount of human DPC having the ability to induce hair follicle-formation to be used for transplant.

Although cells such as DP expressing versican have been shown to specifically have the ability to induce hair follicle-formation under specific conditions (J. Kishimoto, et al., Proc. Natl. Acad. Sci., 96, 7336-7341, 1999), there are many aspects of the phenomenon of hair follicle induction at the molecular level which remain unknown.

## DISCLOSURE OF THE INVENTION

An object of the present invention is to identify factors which regulate hair follicle-formation induction in mouse DPC, which is known to have the ability to induce hair follicle-formation, in order to find factors to enable activation of human DPC by attempting to establish a method for promoting human hair follicle regeneration by controlling the action of molecules regulating hair follicle formation or hair follicle regeneration.

The inventors of the present invention found that, although the ability to induce hair follicle-formation by hair follicle dermal papilla cells is lost as a result of culturing thereof, in the case of culturing those cells at a predetermined high density, the induction ability thereof tends to be retained. When hair follicle dermal papilla cells were cultured under high-density (specifically, 3 to 7 x $10^5$ cells/$cm^2$) and low-density (specifically, 5 to 9 x $10^4$ cells/$cm^2$) conditions to investigate expressed genes, the inventors of the present invention found that expression of the following specific genes is specifically up-regulated in hair follicle dermal papilla cells which were unable to form hair follicles as a result of culturing under low-density conditions, while also found that expression of the following specific genes is specifically up-regulated in hair follicle dermal papilla cells which formed hair follicles as a result of culturing under high-density conditions.

(1) Genes for which expression is specifically up-regulated in hair follicle dermal papilla cells which were unable to form hair follicles as a result of culturing under low-density conditions:

* S100 calcium binding protein A6 gene (S100a6)
* Connective tissue growth factor gene (Ctgf)
* Glutathione-S-transferase omega 1 gene (Gsto1)
* Growth arrest-specific 6 gene (Gas6)
* Kruppel-like factor 2 (Klf2)
* Thrombospongin 1 (Thbs1)
* Thrombomodulin (Thbd)

Thus, it was concluded that these specific genes are intimately involved in inhibition of hair follicle formation and/or regeneration, and that they have the ability to inhibit hair follicle formation, thereby leading to completion of the present invention.
Thus, in a first aspect thereof, the present invention provides a method for regenerating hair follicles by suppressing expression of one or a plurality of genes having the ability to inhibit hair follicle formation selected from the group consisting of S100a6, Ctgf, Gstol, Gas6, Klf2, Thbs1 and Thbd.
In a different mode of this first aspect, the present invention provides a method for culturing hair follicle dermal papilla cells comprising: suppressing the expression of one or a plurality of genes having the ability to inhibit hair follicle formation selected from the group consisting of S10Da6, Ctgf, Gsto1, Gas6, Klf2, Thbs1 and Thbd. Hair follicle dermal papilla cells cultured in this manner can be advantageously used for hair regeneration procedures or hair transplants by transplanting cells to the scalp.
(2) Genes for which expression is specifically up-regulated in hair follicle dermal papilla cells which formed hair follicles as a result of culturing under high-density conditions:

* Transforming growth factor, β-induced, 68 kDa gene (Tgfbi)
* Growth arrest-specific 1 gene (Gas1)
* Thrombospongin 2 gene (Thbs2)
* Interferon-activated gene 202A (If1202A)
* Bone morphogenic protein 7 gene (Bmp7)
* Ephrin A1 gene (Efna1)
* Ephrin A3 gene (Efna3)
* Cell death-inducing DNA fragmentation factor, α-subunit-like effector A gene (Cidea)
* Serine or cysteine proteinase inhibitor, clade G (C1 inhibitor), member 1 gene (Serping1)
* Cysteine proteinase inhibitor 1 gene (MS1)
* Interferon regulatory factor 6 gene (Irf6)
* Fibromodulin gene (Fmod)
* FXYD domain-containing ion transporting regulator 4 gene (Fxyd4)

Thus, it was concluded that these specific genes are intimately involved in induction of hair follicle formation and/or regeneration, and that they have the ability to induce hair follicle formation, thereby leading to completion of the present invention,

Thus, in a second aspect thereof, the present invention provides a method for regenerating hair follicles by up-regulating expression of one or a plurality of genes having the ability to induce hair follicle formation selected from the group consisting of Tgfbi, Gas1, Thbs2, Ifi202A, Bmp7, Efna1, Efna3, Cidea, Serping1, MS1, Irf6, Fmod and Fxyd4.

In a different mode of this second aspect, the present invention provides a method for culturing hair follicle dermal papilla cells comprising : up-regulating the expression of one or a plurality of genes having the ability to induce hair follicle formation selected from the group consisting of Tgfbi, Gas1, Thba2, Ifi202A, Bmp7, Efna1, Efna3, Cidea, serping1, MS1, Irf6, Fmod and Fxyd4. Hair follicle dermal papilla cells cultured in this manner can be advantageously used for hair regeneration procedures or hair transplants by transplanting cells to the scalp.

In addition, in a third aspect thereof, the present invention provides a method for regenerating hair follicles by suppressing the expression of one or a plurality of genes having the ability to inhibit hair follicle formation selected from the group consisting of S100 calcium binding protein A6 gene (S100a6), connective tissue growth factor gene (Ctgf), glutathione-S-transferase omega 1 gene (Gstol), growth arrest-specific 6 gene (Gas6), Kruppel-like factor 2 (Klf2), thrombospongin 1 (Thbs1) and thrombomodulin (Thbd); and, up-regulating the expression of one or a plurality of genes having the ability to induce hair follicle formation selected from the group consisting of transforming growth factor, β-induced, 68 kDa gene (Tgfbi), growth arrest-specific 1 gene (Gas1) thrombospongin 2 gene (Thbs2), interferon-activated gene 202A (Ifi202A), bone morphogenic protein 7 gene (Bmp7), ephrin A1 gene (Efna1), ephrin A3 gene (Efna3), cell death-inducing DNA fragmentation factor, α-subunit effector-like A gene (Cidea), serine or cysteine proteinase inhibitor, clade G (C1 inhibitor), member 1 gene (Serping1), cysteine proteinase inhibitor 1 gene (MS1), interferon regulatory factor 6 gene (Irf6), fibromodulin gene (Fmod) and FXYD domain-containing ion transporting regulator 4 gene (Fxyd4).

In a different mode of this third aspect, the present invention provides a method for culturing hair follicle dermal papilla cells comprising: suppressing the expression of one or a plurality of genes having the ability to inhibit hair follicle formation selected from the group consisting of S100a6, Ctgf, Gsto1, Gas6, Klf2, Thbs1 and Thbd; and, up-regulating the expression of one or a plurality of genes having the ability to induce hair follicle formation selected from the group consisting of Tgfb1, Gas1, Thbs2, Tfi202A, Bmp7, Efna1, Efna3, Cidea, Serping1, MS1, Irf6, Fmod and Fxyd4. Hair follicle dermal papilla cells cultured in this manner can be advantageously used for hair regeneration procedures or hair transplants by transplanting cells to the scalp.

## BRIEF DESCRIPTION OF THE DRAWINGS

FIG. 1 indicates a comparison of mouse hair follicle formation by transplantation of hair follicle dermal papilla cells cultured under high-density (a) and low-density (b) conditions; and,

FIG. 2 indicates the effects of intracutaneous injection of Ctgf on Day 8 (A) and Day 7 (B) of mouse hair growth.

## BEST MODE FOR CARRYING OUT THE INVENTION

As is clear from the results of the examples described to follow, the inventors of the present invention found that the following genes are specifically expressed in hair follicle dermal papilla cells which have lost the ability to induce hair follicle formation and/or regeneration.

* S100a6 (S100 calcium binding protein A6; also known as calciclin)

A calcium binding protein in the form of either S100 protein, calcium-responding protein or calcium sensor protein, which is expressed on the nuclear membrane and is thought to be involved in cell growth by regulating the cell cycle (A. Tomas, et al., J. Biol. Chem., 278, 20210-21216, 2003; E.C. Breen, et al., J. Cell Biochem., 68, 848-854, 2003).

* Ctgf (Connective tissue growth factor)

It has been reported to be secreted as a secreted growth factor in the extracellular matrix, have action which promotes cell adhesion, and be the target gene of induction of fibrosis by TGFβ (A. Leask, et al., J. Biol. Chem., 278, 13008-13015, 2003).

* Gsto1 (Glutathione s-transferase omega 1)

It has glutathione-dependent thiol transferase activity and dehydroascorbic acid reductase activity, and has been suggested to a stress response protein (R. Kodym, et al., J. Biol. Chem., 274, 5131-5137, 1999).

* Gas6 (Growth arrest-specific 6)

A cell growth factor for mesenchymal cells, and is reported to also have a stabilizing action on β-catenins (S. Goruppi, et al., Mol. Cell. Biol., 21, 902-915, 2001; K. Nagai, et al., J. Biol. Chem., 278, 18229-18234, 2003).

* Klf2 (Kruppel-like factor 2)

A transcription factor belonging to the spl family, and is reported to have a function which inhibits differentiation in lipocytes (S.S. Banerjee, et al., J. Biol. Chem., 278, 2581-2584, 2003; J. Kaczynski, et al., Genome Biology, 4, 206.1-206.8, 2003).

* Thbs1 (Thrombospondin 1)

An adhesive glycoprotein which is known to regulate the interaction between cells as well as between cells and the extracellular matrix, and particularly as an endogenous anti-vascularization substance. In addition, it has been reported to be specifically induced in the regression phase of the hair cycle (K. Yano, J. Invest. Dermatol., 120, 14-9, 2003).

* Thbd (Thrombomodulin)

This substance forms a 1:1 complex with thrombin, and demonstrates antiooagulative action by activating protein C as well as anti-fabrotic action by activating procarboxypeptidase B. In addition, it has also been reported to demonstrate anti-inflammatory action by inhibiting adhesion of phlogocytes and vascular endothelial cells (E.M. Conway, et al., J. Exp. Med., 196, 565-577, 2002).

Thus, hair follicle formation and/or regeneration can be induced by suppressing the expression of these genes, thereby enabling hair growth on the scalp hair and other hair. Suppression of these genes can be achieved by applying a drug having such action to scalp hair and so forth. Thus, a composition, and particularly an external preparation for skin, containing such a drug as an active ingredient thereof is expected to demonstrate superior action which promotes hair growth and hair development in humans and other mammals, and is useful as a hair care pharmaceutical, over-the-counter drug or cosmetic.

Suppression of the aforementioned genes in hair follicle dermal papilla cells can be achieved by various genetic engineering technologies using, for example, RNA interference, anti-sense RNA-DNA, peptide and RNA-DNA aptamers, site-specific deletion, homologous recombination, dominant negative alleles or intrabodies.
In a different aspect thereof, as is clear from the results of the examples described to follow, the inventors of the present invention found that the following genes are specifically expressed in hair follicle dermal papilla cells which have the ability to induce hair follicle formation and/or regeneration.
* Tgfbi (Transforming growth factor, beta-induced, 68 kDa)
This secretory protein is also known as βig-h3 (TGF-β-induced gene-human, clone 3) or big-h3. It binds with the extracellular matrix (ECM) such as microfibrils and cell surface collagen through integrin, and together with regulating intercellular adhesion, has also been suggested to be involved in intercellular signal transmission (J.W. Ferguson, et al., Mech. Dev., 120, 851-864, 2003). Although it has been reported to be widely expressed in connective tissue, including the skin and blood vessels (R.G. LeBaron, et al., J. Invest. Dermatol., 104, 844-849, 1995 and the like), expression in hair follicle cells was indicated for the first time by the present invention.
* Gas1 (Growth arrest-specific 1)
It is one of a series of genes (gas) for which expression increases in starving or contact-inhibited 3T3 cells. The product of this gene is a glycoprotein anchored by GPI (glycosyl phosphatidyl inositol) expressed on the cell membrane which inhibits progression of the cell cycle (G. Del Sal, et al., Cell, 70, 595-607, 1992). In addition, it has also been reported to have a function which regulates the expression of FGF10 involved in limb morphogenesis and so forth (Y. Liu, et al., Development, 129, 5289-5300, 2002), and be involved in bone formation through interaction with the ECM (K.K. Lee, et al., Dev. Biol., 234, 188-203, 2001).
* Thbs2 (Thrombospondin 2)
This glycoprotein binds to substances such as fibrinogen, fibronectin, laminin and type V collagen, is involved in interaction between cells as well as between cells and the extracellular matrix, and is also known to have a function which regulates cell growth (N. Lopes, et al., Mol. Cell. Biol., 23, 5401-5408, 2003).
* If1202A (Interferon-activated gene 202A, interferon-inducible p202a)
This is a member of a series of p200-associated proteins induced by interferon which is expressed in the cytoplasm and is known to induce differentiation by migrating into the nucleus and inhibiting transcription factors such as MyoD (H. Xin, et al., Oncogene, 22, 4775-4785, 2003; C. Liu, et al., Mol. Cell. Biol., 20, 7024-7036, 2000).
* Bmp7 (Bone morphogenic protein 7)
This is a secretory protein belonging to the TGF-β family which is generally known to regulate osteogenesis and other morphogenesis. Although it has been confirmed to be expressed in the hair follicle development stage, a direct relationship with an ability to induce hair follicles has yet to be demonstrated, It regulates cell growth and cell differentiation through BMP receptors and various other types of endogenous antagonists (V.A. Botchkarev, J. Invest. Dermatol., 120, 36-47, 2003).
* Efnal (Ephrin A1)
This GPI-anohored protein is expressed on the cell membrane, is a ligand of receptor tyrosine kinase EphA (particularly EphA2), and regulates cell adhesion and cell morphology (C. Deroanne, et al., J. Cell sci., 116, 1367-1376; N. Carter, et al., Nat. Cell Biol., 4, 565-573, 2002).
* Efna3 (Ephrin A3)
This GPI-anchored protein belongs to the family of ephrin proteins, and is a ligand of the receptor tyrosine kinases EphA2 and EphA4. It has been reported to be involved in spinal cord morphogenesis (K.K. Murai, et al., Neurosci., 6, 153-160, 2003).
* Cidea (Cell death-inducing DNA fragmentation factor, alpha subunit-like effector A)
It regulates lipid metabolism and energy balance by being expressed on the mitochondria membrane and so forth in differentiated lipocytes in the form of brown adipose tissue (BAT) (Z. Zhou, et al., Genet., 35, 49-56, 2003). Mice with overexpression of Cidea by genetic engineering techniques have demonstrated caspase-independent induction of cell death (N. Inohara, et al., EMBO J., 17, 2526-2533, 1998).
* Serping1 (Serine or cysteine proteinase inhibitor, clade G (C1 inhibitor), member 1) gene
This is a secretory glycoprotein which is a serpin, the serine proteinase inhibitor super family. It forms a complex with C1r and Cls proteases to inhibit the activity of those proteases. In addition, it is also thought to play an important role in complement activation, coagulation, fibrin lysis and kinin induction (M. Lener, et al., Eur. J. Biochem., 254, 117-122, 1998).
* MS1 (Cysteine proteinase inhibitor)
Also known as Stefin A1, this is a subtype of cystatin-A which has a cysteine protease inhibitory activity (particularly, against cathepsins B, H and L), and is constantly expressed in the epidermis and lymphoid tissue (F.W. Inohara, et al., Genomics, 15, 507-514, 1993; T.A. Korolenko, et al., Bull. Exp. Biol. Med., 136, 46-48, 2003).
* Irf6 (Interferon regulatory factor 6) gene
Although this is a member of an intranuclear transcription factor family in which a DNA binding region is well preserved, the details of its function are unknown. It is expressed most highly in the palate, and is also expressed in the skin and hair follicles. Van der Wonde's syndrome and popliteal pterygium syndrome, which exhibit abnormalities such as cleft palate and skin abnormalities, have been indicated to be caused by a mutation in the SMIR domain (Smad-interferon regulatory factor-binding domain) of the DNA binding site or protein binding site of Irf6 gene, and the regulatory function of Smad-TGF-β signaling has been suggested (S. Kondo, et al., Nat. Gent., 32, 285-289,2002).
* Fmod (Fibromodulin)
This non-collagen secretory protein is a constituent of ECM. It is known to regulate proper fibrogenesis orientation by intereacting with type I and type II collagens as well as inhibit the fibrogenic action of TGF-β (S. Chakravarti, Glycoconj. J., 19, 287-293, 2003; C. Soo, et al., Am. J. Pathol., 157, 423-433, 2000).
* Fxyd4 (FXYD domain-containing ion transporting regulator 4)
It is specifically expressed on the cell membranes of renal epithelial cells, and contributes to electrolyte homeostasis by regulation Na+ and K+ ion transport (R. Aizman, et al., Am. J. Pathol., 283, F569-F577, 2002).

Thus, hair follicle formation and/or regeneration is induced by up-regulating the expression of these genes, thereby enabling hair growth on the scalp and other hair. Up-regulation of these genes can be achieved by applying a drug having such action to the head and so forth. Thus, a composition, and particularly an external preparation for skin, containing such a drug as an active ingredient thereof is expected to demonstrate superior action which promotes hair growth and hair development in humans and other mammals, and is useful as a hair care pharmaceutical, over-the-counter drug or cosmetic.

In addition, up-regulation of the aforementioned genes in hair follicle dermal papilla cells can be achieved by various genetic engineering technologies. For example, when one of the above genes is missing or deleted in hair follicle dermal papilla cells, the expression thereof may be able to be up-regulated by inserting the gene itself into hair follicle dermal papilla cells. In addition, when one of the above genes is present in hair follicle dermal papilla cells but the above gene is defective as a result of being in an inactive or silent state, the expression of that gene can also be up-regulated by introducing a promoter, enhancer or other regulatory sequence which accelerates expression of that gene at a position that enables the regulatory sequence to act on that gene.

Examples of methods which can be applied for inserting the above genes or a promoter or enhancer into cells include gene insertion methods using a virus vector, and non-viral gene insertion methods (Nikkei Science, April 1944, 20-45, Experimental Medicine Special Edition, 12(15), 1994; Experimental Medicine Supplement, "Basic Technology of Gene Therapy", Yodosha Publishing, 1996)). Examples of gene insertion methods using a virus vector include methods in which the above genes are inserted by incorporating a gene into a DNA virus or RNA virus such as retrovirus, adenovirus, adeno-associated virus, herpes virus, vaccinia virus, pox virus, polio virus or simbu virus. Among these, methods using retrovirus, adenovirus, adeno-associated virus or vaccinia virus are particularly preferable. Examples of non-virus gene insertion methods include methods involving direct administration of an expression plasmid (DNA vaccine methods), liposome methods, lipofectin methods, microinjection methods, calcium phosphate methods and electroporation, with DNA vaccine methods and liposome methods being particularly preferable. In addition, methods for allowing the above genes to actually act as pharmaceuticals include in vivo methods, in which DNA is inserted directly into hair follicle dermal papilla cells, and ex vivo methods, in which hair follicle dermal papilla cells are removed from a human subject, DNA is inserted into the cells outside the body, and the cells are then returned to the body (Nikkei Science, April 1994, 20-45; Pharmacology Monthly, 36(1), 23-48, 1994; Experimental Medicine Special Edition, 12(15), 1994), and in vivo methods are used more preferably. In the case of being administered by an in vivo method, DNA is administered directly to an application site such as the location where hair growth is desired to be promoted. Application may be carried out by subcutaneous administration, intracutaneous administration and so forth. In the case of administering by an in vivo method, an injection agent is used typically, and commonly used vehicles may be added as necessary. In addition, in the case of using in the form of liposomes or fusogenic liposomes (such as Sendai virus (HJV) liposomes), the preparation can be in the form of a liposome preparation such as a suspension, cryogen or centrifugal separation-concentrated cryogen.

Expression of a gene having the ability to inhibit hair follicle formation or a gene having the ability to induce hair follicle formation in cells can be determined by, for example, extracting mRNA from the cells and measuring the amount thereof. Extraction and assay of mRNA are known in the art, and quantification of RNA, for example, is carried out by the quantitative polymerase chain reaction (PCR) method. In addition, expression of the gene can be determined by directly measuring the amount of the gene expression product in hair follicle dermal papilla cells. For example, this measurement can be carried out by using a specific antibody to the gene expression product and using a known method in the art, such as an immunostaining method using a fluorescent substance, dye or enzyme. Western blotting method, an immunoassay method such as ELISA or RIA, or various other methods. In addition, the expressed amount of the gene can also be measured by measuring the known biological activity of the gene expression product. In addition, expression of the gene can be determined through in situ hybridization or measuring its other biological activity.

Moreover, the present invention also provides a method for culturing hair follicle dermal papilla cells. This method is comprised of culturing hair follicle dermal papilla cells under conditions which suppress expression of the above genes having the ability to inhibit hair follicle formation, or accelerate the expression of the above genes having the ability to induce hair follicle formation. Since hair follicle dermal papilla cells cultured in this manner have profound hair follicle regeneration and/or formation ability, they can be used advantageously for hair follicle and/or hair regeneration and transplant by cell transplantation. As was mentioned at the outset, although it was difficult in the prior art to acquire human hair follicle dermal papilla cells having the ability to induce hair follicles in an adequate amount so as to enable the use thereof in transplantation, if hair follicle dermal papilla cells are cultured according to the present invention, hair follicle dermal papilla cells having the ability to induce hair follicles can be prepared in an adequate amount for transplantation from even a small amount of hair follicle dermal papilla cells. As was previously described, by culturing in the presence of a drug which suppresses the expression of the aforementioned genes having the ability to inhibit hair follicle formation, or by transforming using genetic engineering technologies as was previously described, hair follicle dermal papilla cells can be cultured in which the expression of the genes is suppressed. In addition, by culturing in the presence of a drug which up-regulates the expression of the aforementioned genes having the ability to induce hair follicle formation, or by transforming using genetic engineering technologies as previously described, hair follicle dermal papilla cells can be cultured in which the expression of the genes is up-regulated. Culturing is carried out in a suitable medium such as DMEM preferably in a $CO_2$ environment at room temperature to about 37°C, and preferably at about 37°C, for 1 to 7 days.

"Hair follicle dermal papilla cells" refer to mesenchymal cells which primarily compose hair papilla located within the hair bulb portions of hair follicles. These cells fulfill the role of a kind of "control tower" which sends out activation signals to hair follicle epithelial cells and other cells for self-regeneration of hair follicles (Japanese Patent Application No. 2003-346937). Hair follicle dermal papilla cell preparations containing only activated hair follicle dermal papilla cells are described in, for example, Kishimoto, et al., Proc. Natl. Acad. Sci. USA, 96, 7336-7341, 1999, and can be prepared using transgenic mice. However, in terms of yield, however, these cells can be preferably prepared by removing epidermal tissue from skin tissue, treating the resulting dermal tissue fraction with collagen to prepare a cell suspension, and then cryopreserve the cell suspension to damage the hair follicle epithelial cells,

specific examples of methods using cryopreservation as described above can be carried out as indicated below.

1. Prepare mammalian epidermis.
2. Allow the epidermis to stand undisturbed for a suitable amount of time such as overnight in a protease solution such as a trypsin solution as necessary, remove the epidermal portion with a forceps and so forth, and treat the remaining dermis with collagenase to prepare a cell suspension.
3. Filter the cell suspension through a strainer as necessary and remove the precipitate by allowing to stand undisturbed.
4. Measure the number of cells, resuspend the cells in a cryoprotectant liquid at a suitable cell density of preferably about $1 \times 10^3$ to $1 \times 10^8$ cells/ml, apportion into smaller aliquots as necessary, and then place in frozen storage in accordance with ordinary cell storage methods.
5. After storing for a suitable period of time, thaw the cells for use.

Although there are no particular limitations on the freezing method, the cells are stored in an ultra-low-temperature freezer or in liquid nitrogen at a temperature of -20°C or lower, preferably -50°C or lower and more preferably at -80°C or lower. Although there are also no particular limitations on the duration of storage, the cells are stored for, for example, 1 day or more, preferably 3 days or more, and more preferably 1 week or more, to ensure cell death of the epithelial cells. Furthermore, hair follicle dermal papilla cells have been confirmed to remain viable even if stored for 4 months in liquid nitrogen. Ordinary preservatives such as Cell Banker 2 Cell Frozen Storage Liquid (Cat. No. BLC-2, Nippon Zenyaku Kogyo) can be used for the cryoprotectant liquid.

The hair follicle dermal papilla cells of the present invention can be derived from the epidermis of any mammals, including humans, chimpanzees and other primates, domestic animals such as dogs, cats, rabbits, horses, sheep, goats, cows and pigs, as well as laboratory animals such as rats, mice and guinea pigs, and are preferably derived from nude mice, acid mice and nude rats.

Hair follicle dermal papilla cells acquired in this manner can be used to transplant to the hair a mixture of these cultured hair follicle dermal papilla cells and epithelial cells by culturing under conditions which suppress the expression of the aforementioned genes having the ability to inhibit hair follicle formation or under conditions which accelerate the expression of the aforementioned genes having the ability to induce hair follicle formation according to the present invention, followed by mixing with suitable epithelial cells.

"Epithelial cells" refers to those cells which compose the majority of the epidermis or epithelium of skin, and arise from a layer of basal cells in contact with the dermis. In using the example of mice, although epithelial cells derived from newborns (or fetuses) can be preferably used as epithelial cells, cells derived from the epidermis of aged skin as well as cultured cells in the form of keratinocytes can also be used. These cells can be prepared from the skin of a desired donor animal according to methods known among persons skilled in the art.

In a preferable aspect thereof, epithelial cells can be prepared in the manner described below.

1. Prepare mammalian epidermis.
2. Treat this epidermis with trypsin by allowing to stand undisturbed overnight at 4°C in 0.25% trypsin in PBS as necessary.
3. Separate the epidermal portion with a forceps and so forth, and after cutting into thin sections, suspend for about 1 hour in a suitable culture medium (for example, keratinocyte culture medium) at 4°C.
4. Filter the suspension through a strainer having a suitable pore size, and then apply to a centrifuge to retrieve the epithelial cells.
5. This cell preparation is then suspended at a desired cell density in KGM or SFM medium, and allowed to stand undisturbed on ice until just prior to use.

Similar to the aforementioned hair follicle dermal papilla cells, epithelial cells of the present invention can be derived from the epidermis of any mammals, including humans, chimpanzees and other primates, domestic animals such as dogs, cats, rabbits, horses, sheep, goats, cows and pigs, as well as laboratory animals such as rats, mice and guinea pigs, and are preferably derived from nude mice, scid mice and nude rats. In addition, the site of the epidermis may be a hairy site such as the scalp or a glabrous site such as the foreskin.

The ratio of the number of cultured hair follicle dermal papilla cells to the number of epithelial cells is 1:3 to 10: 1, preferably 1:1 to 10:1, more preferably 1:1 to 3:1 and most preferably 1:1 to ensure hair follicle formation.

The hair follicle dermal papilla cells and epithelial cells of the same species or different species may be combined. For example, in the case a hair follicle dermal papilla cell preparation is derived from mice, the epithelial cells may be derived from mice (combination of same species), or they may be derived from another species such as rats or humans (different species). Thus, a composition for hair transplant may be, for example, a combination of cultured hair follicle dermal papilla cells and epithelial cells both derived from mice, a combination thereof both derived from rats, or a combination thereof both derived from humans (same species), or a combination in which cultured hair follicle dermal papilla cells are derived from mice and epithelial cells are derived from rats, a combination in which cultured hair follicle dermal papilla cells are derived from rats and epithelial cells are derived from mice, a combination in which cultured hair follicle dermal papilla cells are derived from mice and epithelial cells are derived from humans, a combination in which cultured hair follicle dermal papilla cells are derived from rats and epithelial cells are derived from humans, a combination in which cultured hair follicle dermal papilla cells are derived from humans and epithelial cells are derived from mice, or a combination in which cultured hair follicle dermal papilla cells are derived from humans and epithelial cells are derived from rats (xenotypic combination).

The following provides a more detailed explanation of the present invention through examples thereof.

Preparation of Hair Follicle Dermal Papilla Cells

After washing newborn ICR mice with ethanol and phosphate-buffered saline (PBS), the dorsal skin was excised and the whole skin was obtained. After floating the skin in a trypsin solution and allowing to stand undisturbed overnight at 4°C, the epidermis was removed with a forceps, and the resulting dermis was treated with collagenase to obtain a cell suspension. The suspension was filtered with a cell strainer followed by removing the precipitate by allowing to stand undisturbed overnight to obtain a DP fraction. The cells were resuspended in a cryoprotectant liquid to a cell density of $1 \times 10^5$ to $1 \times 10^8$ cells/ml, the suspension was apportioned into freezing tubes, the cells were frozen for 1 week or more in liquid nitrogen in accordance with ordinary cell freezing methods. The cells were then thawed in DMEM (10% FBS) and cultured in DMEM (10% FBS) at a high density (3 to $7 \times 10^5$ cells/$cm^2$) or a low density (5 to $9 \times 10^4$ cells/$cm^2$) for 1 to 4 days in a $CO_2$ environment at 37°C.

preparation of Epithelial Cell Fraction

After washing newborn ICR mice with ethanol and PBS, skin was removed from the back and the whole skin were excised. After floating the skin in a trypsin solution and allowing to stand undisturbed overnight at 4°C, the epidermis was removed with a forceps, cut into thin sections, and stirred and suspended for about 1 hour in keratinocyte culture medium (SFM) at 4°C. After removing solid matter with a cell strainer, the filtrate was subjected to centrifugal separation (x 900 g, 10 minutes) and the resulting pellet was resuspended in SFM medium to obtain an epithelial cell fraction. This fraction was allowed to stand undisturbed on ice until just before use.

Hair Follicle Regeneration Evaluation System by Transplanting Cells to Skin on the Back of Nude Mice

A DP fraction prepared by culturing for 4 days in the manner described above (high-density culture or low-density culture: 5 to $10 \times 10^6$ cells) and an epithelial fraction (from 2 animals, about $1 \times 10^7$ cells) were mixed gently followed by centrifugation, removal of the supernatant, and seeding the resulting pellet onto a muscle membrane inside a silicon chamber surgically implanted in advance in a nude mouse. One week later, the upper portion of the silicon chamber was cut away, and two weeks later, the silicon chamber was removed. Hair follicle formation was assessed by observing appearance and tissue 3 to 4 weeks after cell transplant. Those results are shown in FIG. 1.

As is clear from FIG. 1, although hair growth was observed and hair follicles had formed in the case of having transplanted hair follicle dermal papilla cells cultured under high-density conditions, hair growth was not observed and hair follicles were verified not to be formed in the case of having transplanted hair follicle dermal papilla cells cultured under low-density conditions. Thus, culturing of hair follicle dermal papilla cells under conditions of a high cell density of a predetermined level or more was clearly determined to be required for forming hair follicles by transplanting cultured hair follicle dermal papilla cells.

Microarray Experiment

1 ml of Isogen (Nippon Gene) was added to a DP fraction prepared by culturing for 1 or 4 days as previously described (high-density culturing or low-density culturing: 5 to 10 x $10^6$ cells), followed by extraction of 50 to 100 μg of total RNA according to the manual. Moreover, the resulting total RNA was purified according to the RNeasyMini (Qiagen) RNA Cleanup method, and the absence of contamination by degradation products was confirmed with the Bioanalyzez 2100 System (Agilent). From 500 ng of the resulting total RNA, fluorescent dye-labeled cRNA was prepared with cy5-CTP or Cy3-CTP (Perkin-Elmer) using Low RNA Input Fluorescent Lamp Amplification (Agilent) in accordance with its manual (for example, RNA derived from high-density cultured DF was labeled with Cy5, while RNA derived from low-density cultured DP was labeled with Cy3). 1 μg of each fluorescent dye-labeled cRNA was subjected to competitive hybridization for 17 hours at 60°C on a Mouse Development Oligo Microarray (Agilent G4120A) or Mouse Oligo Microarray (Agilent G4121A) slide using Hybridization Kit Plus (Agilent) in accordance with its manual. After washing and drying, the mioroarrays were immediately converted to images with a microarray scanner (Agilent G2565AA). The resulting images were quantified for the fluorescent intensity of each spot using Feature Extraction software (Agilent G2566AA), and used for analysis. The expressed amount of each gene is shown in the tables below.

Table 1

| Gene | | 1 day of culture | | | | 4 days of culture | | | |
|---|---|---|---|---|---|---|---|---|---|
| | | Low density | High density | High/ low ratio | Array | Low density | High density | High/ low ratio | Array |
| S100a6 | 1st | 488337 | 307137 | 0.63 | (1) | 145980 | 87903 | 0.60 | (2) |
| | 2nd | 329059 | 220925 | 0.67 | (1) | 317877 | 125517 | 0.39 | (1) |
| | | | | | | 216505 | 121178 | 0.56 | (2) |
| Ctgf | 1st | 86260 | 9880 | 0.11 | (1) | 79019 | 45124 | 0.57 | (2) |
| | 2nd | 74757 | 21760 | 0.29 | (1) | 65201 | 19354 | 0.30 | (1) |
| | | | | | | 155952 | 83250 | 0.53 | (2) |
| Gsto1 | 1st | 34031 | 27582 | *0.01 | (1) | 61350 | 29066 | 0.49 | (2) |
| | 2nd | 24630 | 12764 | 0.52 | (1) | 21169 | 18076 | 0.85 | (1) |
| | | | | | | 38542 | 21886 | 0.57 | (2) |
| Gas6 | 1st | 5148 | 8136 | 1.58 | (1) | 33250 | 12622 | 0.38 | (2) |
| | 2nd | 10344 | 23361 | 2.26 | (1) | 13552 | 6920 | 0.51 | (1) |
| | | | | | | 22655 | 9511 | 0.42 | (2) |
| Klt2 | 1st | 23433 | 8251 | 0.35 | (1) | 8931 | 3487 | 0.39 | (2) |
| | 2nd | 15628 | 5425 | 0.35 | (1) | 5417 | 4010 | 0.74 | (1) |
| | | | | | | 2861 | 1762 | 0.62 | (2) |
| Thbs1 | 1st | 4298 | 2794 | 0.65 | (1) | 3274 | 3094 | 0.94 | (2) |
| | 2nd | 6817 | 3063 | 0.45 | (1) | 6598 | 4081 | 0.62 | (1) |
| | | | | | | 6186 | 5665 | 0.92 | (2) |

(continued)

| Gene | | 1 day of culture | | | | 4 days of culture | | | |
|---|---|---|---|---|---|---|---|---|---|
| | | Low density | High density | High/ low ratio | Array | Low density | High density | High/ low ratio | Array |
| Thbd | 1st | 1287 | 907 | 0.70 | (1) | 1386 | 1793 | 1.29 | (2) |
| | 2nd | 690 | 272 | 0.39 | (1) | 592 | 317 | 0.53 | (1) |
| | | | | | | 2840 | 1415 | 0.50 | (2) |
| (1) Using Mouse Development Oligo Microarray<br>(2) Using Mouse Oligo Microarray * No significant difference | | | | | | | | | |

Table 2

| Gene | | 1 day of culture | | | | 4 days of culture | | | |
|---|---|---|---|---|---|---|---|---|---|
| | | Low density | High density | High/ low ratio | Array | Low density | High density | High/ low ratio | Array |
| Tgfbi | 1st | 2825 | 6673 | 2.36 | (1) | 17533 | 43006 | 2.45 | (2) |
| | 2nd | 4589 | 11397 | 2.48 | (1) | 4087 | 8434 | 2.06 | (1) |
| | | | | | | 6671 | 23863 | 2.75 | (2) |
| Gas1 | 1st | 6407 | 32952 | 5.14 | (1) | 4792 | 7182 | 1.50 | (2) |
| | 2nd | 8852 | 30016 | 3.39 | (1) | 10524 | 21235 | 2.02 | (1) |
| | | | | | | 3152 | 5020 | 1.59 | (2) |
| Thbs2 | 1st | 11672 | 34827 | 2.98 | (1) | 27163 | 28831 | *1.04 | (2) |
| | | 2819 | 14396 | 5.11 | | | | | |
| | 2nd | 23664 | 65034 | 2.75 | (1) | 12554 | 21480 | 1.71 | (1) |
| | | 2986 | 6287 | 2.12 | | 3296 | 6698 | 2.03 | |
| | | | | | | 9931 | 16898 | 1.89 | (2) |
| If1202a | 1st | 2409 | 41216 | 17.11 | (1) | 16867 | 23814 | 1.41 | (2) |
| | 2nd | 4162 | 17194 | 4.13 | (1) | 2376 | 3594 | 1.51 | (1) |
| | | | | | | 5853 | 7684 | *1.31 | (2) |
| (1) Using Mouse Development Oligo Microarray<br>(2) Using Mouse Oligo Microarray * No significant difference | | | | | | | | | |

Table 3

| Gene | | 1 day of culture | | | | 4 days of culture | | | |
|---|---|---|---|---|---|---|---|---|---|
| | | Low density | High density | High/ low ratio | Array | Low density | High density | High/ low ratio | Array |
| Bmp7 | 1st | 1362 | 4916 | 3.61 | (1) | 1224 | 3238 | 2.65 | (2) |
| | 2nd | 1307 | 4134 | 3.16 | (1) | 624 | 1325 | 2.12 | (1) |
| | | | | | | 645 | 1239 | 1,92 | (2) |
| Efna1 | 1st | 3577 | 9031 | 2.52 | (1) | 808 | 1511 | 1.87 | (2) |
| | 2nd | 2970 | 8976 | 3.01 | (1) | 1295 | 3610 | 2.79 | (1) |
| | | | | | | 461 | 844 | 1,83 | (2) |

(continued)

| Gene | | 1 day of culture | | | | 4 days of culture | | | |
|---|---|---|---|---|---|---|---|---|---|
| | | Low density | High density | High/ low ratio | Array | Low density | High density | High/ low ratio | Array |
| Efna3 | 1st | | | | | 446 | 821 | 1.84 | (2) |
| | 2nd | | | | | 279 | 533 | 1.91 | (2) |
| Cidea | 1st | 405 | 1229 | 3.04 | (1) | 1271 | 6124 | 4.02 | (2) |
| | 2nd | 564 | 1308 | 2.32 | (1) | 186 | 551 | 2.96 | (1) |
| | | | | | | 393 | 1386 | 3.53 | (2) |
| Serping1 | 1st | 3495 | 17368 | 4.97 | (1) | 723 | 1943 | 2.69 | (2) |
| | 2nd | 5187 | 31137 | 6.00 | (1) | 2818 | 3907 | 1.39 | (1) |
| | | | | | | 471 | 738 | 1.57 | (2) |
| MS1 | 1st | 542 | 5597 | 10.32 | (1) | 1136 | 3507 | 3.09 | (2) |
| | 2nd | 834 | 5326 | 6.39 | (1) | 173 | 374 | 2,16 | (1) |
| | | | | | | 604 | 998 | 1.65 | (2) |
| Irf6 | 1st | 664 | 789 | *1.29 | (1) | 5282 | 9098 | 1.72 | (2) |
| | 2nd | 517 | 903 | 1.75 | (1) | 583 | 690 | 1.18 | (1) |
| | | | | | | 2230 | 3264 | 1.46 | (2) |
| Fmod | 1st | 317 | 433 | *1.37 | (1) | 2979 | 3127 | *1.05 | (2) |
| | 2nd | 1144 | 2521 | 2.20 | (1) | 16600 | 24305 | 1.46 | (1) |
| | | | | | | 3303 | 4853 | 1.47 | (2) |
| Fxyd4 | 1st | 252 | 654 | 2.59 | (1) | 412 | 1023 | 2.48 | (2) |
| | 2nd | 192 | 395 | 2.06 | (1) | 512 | 674 | *1.32 | (1) |
| | | | | | | 462 | 650 | 1.41 | (2) |
| (1) Using Mouse Development Oligo Microarray<br>(2) using Mouse Oligo Microarray<br>* No significant difference | | | | | | | | | |

On the basis of the above results, the expressed amount of a specific gene was clearly determined to vary depending on whether hair follicle dermal papilla cells are cultured under high-density conditions or cultured under low-density conditions. Namely, with respect to genes S100a6, Ctgf, Gsto1, Gas6, Klf2, Thbs1 and Thbd, culturing hair follicle dermal papilla cells under low-density conditions at which hair follicle are not formed was clearly demonstrated to increase the expressed amount of each gene as compared with culturing under high-density conditions, and those genes were thought to have action which inhibits hair follicle formation. In addition, with respect to genes Tgfbi, Gas1, Thbs2, If1202A, Bmp7, Efna1, Efna3, Cidea, Serping1, M51, Irf6, Fmod and Fxyd4, culturing hair follicle dermal papilla calls under high-density conditions which enabled hair follicle formation was clearly demonstrated to increase the expressed amount of each gene as compared with culturing under low-density conditions, and those genes were thought to be intimately involved in hair follicle formation and regeneration, and have the ability to induce hair follicle formation.

<u>Quantitative PCR Experiment</u>

After removing genomic DNA by DNase treatment (DNA-free RNA Kit™, Zymo Research), the purified total RNA prepared for the aforementioned microarray experiment was used to synthesize cDNA with SuperScriptII (Invitrogen) using random primers (Pharmacia). Quantification was then carried out by real-time PCR using CyberGreen according to the manual using this cDNA for the template and using the LightCycler-FastStart DNA Master-SYER Green I Kit with LightCycler (both from Roche) (final concentration of $Mg^{2+}$: 3 mM, other reaction conditions were as described below). GAPDH (size of PCR product: 201 pb) final concentration: 0.25 $\mu$M

Forward: 5'-GAGTCAACGGATTTGGTCGT-3' (NM002046: 95-104) (SEQ ID NO. 1)
Reverse: 5'-TGGGATTTCCATTGATGACA-3' (NM002046: 295-276 (SEQ Ib NO. 2)
40 cycles of denaturation at 95°C for 15 seconds, annealing at 55°C for 10 seconds and elongation for 10 seconds
Serping1 (size of PCR product: 430 pb, Lener, M. et al., 1998) final concentration : 0.5 μM
Forward: 5'-GAATTCTTTGACTTCACTTA-3' (NM_009776 : 1327-1346) (SEQ ID NO. 3)
Reverse : 5'-ATTTGTAGAGTTTGATAGGT-3' (NM_009776 : 1765-1746 (SEQ ID NO. 4)
40 cycles of denaturation at 95°C for 15 seconds, annealing at 55°C for 10 seconds and elongation at 72°C for 20 seconds
Efna1 (size of PCR product: 133 pb, Pickles, J.O. et al., 2003) final concentration: 0.5 μM
Forward : 5'-TCTGGGCAGTATTGCTCCTAC-3' (NM_010107: 672-692) (SEQ ID NO. 5)
Reverse: 5'-CTTGTGGGTGTAGTGGGAGAG-3' (NM_010107: 804-784). (SEQ ID NO. 6)
40 cycles of denaturation at 95°C for 15 seconds, annealing at 55°C for 10 seconds and elongation at 72°C for 10 seconds
Efna3 (size of PCR product: 171 pb, Pickles, J.O. et al., 2003) final concentration: 0.5 μM
Forward: 5'-TATTTGTCCGCACTACAACAG-3' (MMU90666: 8-28) (SEQ ID NO. 7)
Reverse: 5'-AATTTTTCGGAGAACTTGATG-3' (MMU90666: 178-158) (SEQ ID NO. 8)
40 cycles of denaturation at 95°C for 15 seconds, annealing at 58°C for 5 seconds and elongation at 72°C for 10 seconds
Gas1 (size of PCR product: 205 pb) final concentration: 0.5 μM
Forward : 5'-GGGGTCTTTCAAGTTCCAAT-3' (NM_008086 : 1868-1887) (SEQ ID NO. 9)
Reverse: 5'-TCGGTAAGGGGAACTTTTCT-3' (NM_008086 : 2072-2053) (SEQ ID NO. 10)
40 cycles of denaturation at 95°C for 15 seconds, annealing at 55°C for 10 seconds and elongation at 72°C for 10 seconds
Conversion of Amount of Target Gene Corrected with GAPDH:
Quantification according to the Fit Point method was used by logarithmically plotting the fluorescent luminosity of the CyberGreen measured during the elongation of the PCR cycle versus the number of cycles, setting the threshold level, and defining the intersection with the logarithmic linear amplification region of each sample as the number of cycles in which the signal rose (crossing point).

When the amount of PCR product (namely, fluorescent luminosity) is taken to be Y, the initial amount of template is taken to be A, the factor relating to PCR efficiency is taken to be B (provided that $0.5 < B \leq 1$), and the number of cycles is taken to be A, then the following equation results:

$$Y = A \times (B \times 2)^X$$

The logarithm of the right side of this equation is then taken to express the equation as follows:

$$Y = \log_{10}[A(B \times 2)^X] = \log_{10}A + \log_{10}(B \times 2)^X$$
$$= X \times \log_{10}(B \times 2) + \log_{10}A$$

In the region where PCR proceeds theoretically, namely the region in which the logarithmic plot is linear, B = 1, and the slope is constant even for PCR using a different sample for the template. When the threshold (noise band) of the fluorescence value is set (based on Y - C), and the intersection with this value (crossing point) is determined, the number of rise cycles is obtained.

$$A = 10^{(C - X\log_{10}2)}$$

This reflects the initial amount of template. Thus, if the PCR efficiency is the same, the amount of template of a different sample is based on the amount of template $A_0$ of any one sample, and the relative value $A/A_0$ is determined from the respective number of rise cycles ($X, X_0$) .

$$X_0 - X = (C - \log_{10}A_0)/\log_{10}2 - (C - \log_{10}A)/\log_{10}2$$
$$\log_{10}2 \times (X_0 - X) = (C - \log_{10}A_0) - (C - \log_{10}A) = \log_{10}A - \log_{10}A_0$$
$$\log_{10} 2^{(X_0 - X)} = \log_{10}(A / A_0)$$
$$A / A_0 = 2^{(X_0 - X)}$$

The value resulting from standardizing the initial amount of template ($A/A_0$) of a target gene (rabp4, Fmod, serg1, Efna1, Efna3) determined in this manner with the initial amount of template of the GAPDH of each sample ($A_G/A_{G0}$) :

$$((A/A_0)/(A_G/A_{G0}) = 2^{(X_0-X)}/2^{(X_{G0}-X_G)})$$

was compared for each reaction condition (culturing for 1 or 4 days under high-density or low-density conditions). Those results are shown in the table below.

Table 4

| Summary of Quantitative PCR Results (Comparison of High-Density Culturing/Low-Density Culturing) | | | | |
|---|---|---|---|---|
| Gene | 1 day of culture (1st) | 1 day of culture (2nd) | 4 days of culture (1st) | 4 days of culture (2nd) |
| Serg1 | 12.91 | 43.87 | 4.21 | 1.38 |
| Efna1 | 1.81 | 5.92 | 2.41 | 3.81 |
| Efna3 | 3.20 | 6.57 | 3.11 | 6.34 |
| Gael | 7.44 | 8.46 | 1.60 | 2.41 |

Study of the Effects of Hair Follicle Formation Inhibitory Factor on Mouse Hair Growth

Ctgf, which is predicted to have the ability of inhibiting hair follicle formation on the basis of the above experiments, was injected into mouse skin followed by confirmation of inhibition of hair growth.

Experiment Method:

[Day -2]

The backs of twelve 8-week-old C57BL/6 mice (3 groups of 4 animals each) were shaved, and a mark (tattoo) was made near the center of the back. Connective tissue growth factor (Ctgf, Biovendor Laboratory Medicine, Inc.) dissolved in 0.1% BSA-PBS was injected into the skin at the marked locations at 0 (control), 100 or 333 ng/100 μl.

[Day -1]

Ctgf dissolved in 0.1% BSA-PBS was again injected into the skin at the marked locations on the mice at 0 (control), 100 or 333 ng/100 μl in the same manner as on Day -2.

[Day 0]

Hair at the marked locations on the mice was removed by waxing and Ctgf dissolved in 0.1% BSA-PBS was injected into the skin at the marked locations on the mice at 0 (control), 100 or 333 ng/100 μl in the same manner as on Day -1.

[Day +1]

Ctgf dissolved in 0.1% BSA-PBS was injected into the skin at the locations where the hair had been removed by waxing at 0 (control), 100 or 333 ng/100 μl in the same manner as on Day -1.

[Days +4 to +8]

The status of hair growth at the locations where the hair had been removed by waxing was visually observed, and blackening of the skin on the back was measured with a Hexameter (narrow-band reflectance spectrophotometer (Mexameter 160, Courage + Khazaka Electric GmbH, Kern, Germany) to evaluate the degree of hair follicle formation.

Experiment Results:

As a result of intracutaneously injecting Ctgf directly into the backs of mice subjected to hair removal by waxing, hair growth and hair follicle formation were observed to be significantly delayed dependent on the injected amount both visually and by measuring with a Mexameter. The results for Day +7 and Day +8 are shown in Fig. 2. Thus, Ctgf was confirmed to have the ability to inhibit hair follicle formation.

## INDUSTRIAL APPLICABILITY

According to the present invention, methods for growing and transplanting hair can be provided which are both novel and advantageous as compared with conventional technologies.

## Claims

1. A method for regenerating hair follicles by suppressing expression of one or a plurality of genes having the ability to inhibit hair follicle formation selected from the group consisting of S100 calcium binding protein A6 gene (S100a6), connective tissue growth factor gene (Ctgf), glutathione-S-tranaferase omega 1 gene (Gsto1), growth arrest-specific 6 gene (Gas6), Kruppel-like factor 2 (Klf2), thrombospongin 1 (Thbs1) and thrombomodulin (Thbd).

2. A method for culturing hair follicle dermal papilla cells comprising: suppressing the expression of one or a plurality of genes having the ability to inhibit hair follicle formation selected from the group consisting of S100a6, Ctgf, Gsto1, Gas6, Klf2, Thbs1 and Thbd.

**3.** A method for regenerating hair follicles by up-regulating expression of one or a plurality of genes having the ability to induce hair follicle formation selected from the group consisting of transforming growth factor, β-induced, 68 kDa gene (Tgfbi), growth arrest-specific 1 gene (Gas1), thrombospongin 2 gene (Thbs2), interferon-activated gene 202A (Ifi202A), bone morphogenic protein 7 gene (Bmp7), ephrin A1 gene (Efna1), ephrin A3 gene (Efna3), cell death-inducing DNA fragmentation factor, α-subunit-like effector A gene (Cidea), serine or cysteine proteinase inhibitor, clade G (Cl inhibitor), member 1 gene (Serping1), cysteine proteinase inhibitor 1 gene (MS1), interferon regulatory factor 6 gene (Irf6), fibromodulin gene (Fmod) and FXYD domain-containing ion transporting regulator 4 gene (Fxyd4).

**4.** A method for culturing hair follicle dermal papilla cells comprising: up-regulating the expression of one or a plurality of genes having the ability to induce hair follicle formation selected from the group consisting of Tgfbi, Gas1, Thbs2, Ifi202A, Bmp7, Efna1, Efna3, Cidea, Serping1, MS1, Irf6, Fmod and Fxyd4.

**5.** A method for regenerating hair follicles by suppressing expression of one or a plurality of genes having the ability to inhibit hair follicle formation selected from the group consisting of S100 calcium binding protein A6 gene (S100a6), connective tissue growth factor gene (Ctgf), glutathione-S-transferase omega 1 gene (Gsto1), growth arrest-specific 6 gene (Gas6), Kruppel-like factor 2 (Klf2), thrombospongin 1 (Thbs1) and thrombomodulin (Thbd), and up-regulating expression of one or a plurality of genes having the ability to induce hair follicle formation selected from the group consisting of transforming growth factor, β-induced, 68 kDa gene (Tgfbi), growth arrest-specific 1 gene (Gas1), thrombospongin 2 gene (Thbs2), interferon-activated gene 202A (If1202A), bone morphogenic protein 7 gene (Bmp7), ephrin A1 gene (Efna1), ephrin A3 gene (Efna3), cell death-inducing DNA fragmentation factor, α-subunit-like effector A gene (Cidea), serine or cysteine proteinase inhibitor, clade G (C1 inhibitor), member 1 gene (Serping1), cysteine proteinase inhibitor 1 gene (MS1), interferon regulatory factor 6 gene (Irf6), fibromodulin gene (Fmod) and FXYD domain-containing ion transporting regulator 4 gene (Fxyd4).

**6.** A method for culturing hair follicle dermal papilla cells comprising: suppressing the expression of one or a plurality of genes having the ability to inhibit hair follicle formation selected from the group consisting of S100a6, Ctgf, Gsto1, Gas6, Klf2, Thbs1 and Thbd, and up-regulating the expression of one or a plurality of genes having the ability to induce hair follicle formation selected from the group consisting of Tgfbi, Gas1, Thbs2, If1202A, Bmp7, Efna1, Efna3, Cidea, Serping1, MS1, Irf6, Fmod and Fxyd4.

Fig.1

(a) (b)

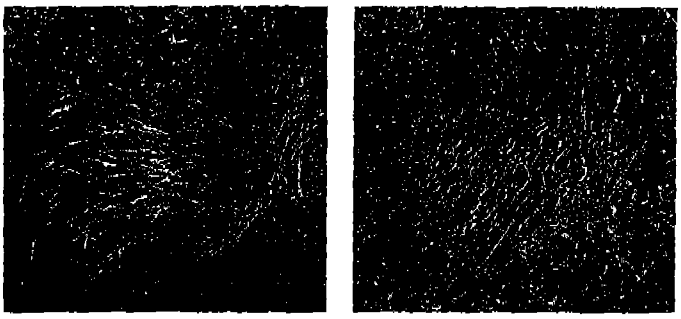

Fig.2

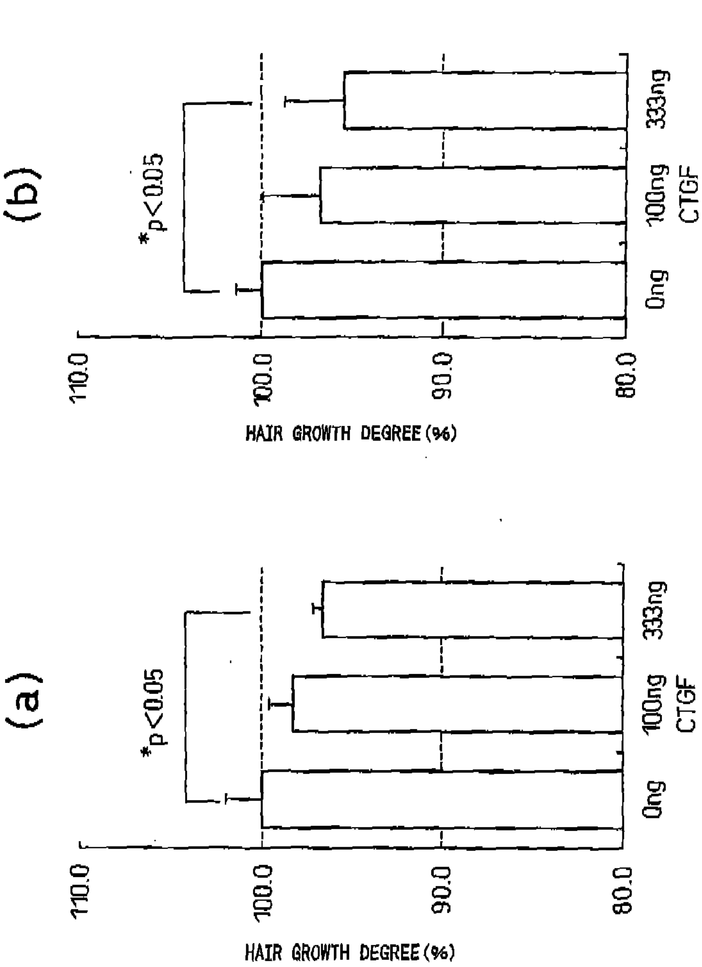
(a)
*p<0.05
HAIR GROWTH DEGREE (%)
110.0
100.0
90.0
80.0
0ng
100ng
333ng
CTGF
(b)
*p<0.05
HAIR GROWTH DEGREE (%)
110.0
100.0
90.0
80.0
0ng
100ng
333ng
CTGF

## INTERNATIONAL SEARCH REPORT

International application No.
PCT/JP2005/011094

A. CLASSIFICATION OF SUBJECT MATTER
Int.Cl[7] A61K45/00, A61P17/14, C12N5/10, 15/09

According to International Patent Classification (IPC) or to both national classification and IPC

B. FIELDS SEARCHED

Minimum documentation searched (classification system followed by classification symbols)
Int.Cl[7] A61K45/00, A61P17/14, C12N5/10, 15/09

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
BIOSIS(STN), CAplus(STN), EMBASE(STN), MEDLINE(STN), JSTPlus(JOIS)

C. DOCUMENTS CONSIDERED TO BE RELEVANT

| Category* | Citation of document, with indication, where appropriate, of the relevant passages | Relevant to claim No. |
|---|---|---|
| A | Mutsumi INAMATSU et al., "Mo'nyuto Saibo o Mochiita Mohatsu Saisei", Saisei Iryo, 2002, Vol.2, No.3, pages 114 to 119 | 2,4,6 |
| A | Tomoyo TATENO et al., "Zoki Saisei to Kansaibo Seibutsugaku, Hifu to Kanzo no Saisei", Iden Separate Volume, 2001, Vol.13, pages 53 to 61 | 2,4,6 |
| A | Shigeki INUI et al., "Saikin no Datsumo Ikumo Mechanism Kenkyu to Ikumozai no Kaihatsu Doko", Fragrance Journal, 2003, Vol.31, No.2, pages 9 to 14 | 2,4,6 |
| A | WO 2003/051419 A (Henkel KgaA), 26 June, 2003 (26.06.03), & EP 1455854 A1 & US 2005/089512 A1 & DE 10162814 A1 | 2,4,6 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>13 September, 2005 (13.09.05) | Date of mailing of the international search report<br>11 October, 2005 (11.10.05) |
| Name and mailing address of the ISA/<br>Japanese Patent Office<br>Facsimile No. | Authorized officer<br><br>Telephone No. |

Form PCT/ISA/210 (second sheet) (January 2004)

INTERNATIONAL SEARCH REPORT

International application No.
PCT/JP2005/011094

C (Continuation). DOCUMENTS CONSIDERED TO BE RELEVANT

| Category* | Citation of document, with indication, where appropriate, of the relevant passages | Relevant to claim No. |
|---|---|---|
| A | JP 2001-302520 A (Akimasa TAKAHASHI et al.), 31 January, 2001 (31.01.01), (Family: none) | 2,4,6 |
| A | JP 9-140377 A (Shiseido Co., Ltd.), 03 June, 1997 (03.06.97), (Family: none) | 2,4,6 |
| A | JP 7-274950 A (Research Development Corp. of Japan et al.), 24 October, 1995 (24.10.95), & US 5851831 A & EP 682107 A3 | 2,4,6 |

Form PCT/ISA/210 (continuation of second sheet) (January 2004)

INTERNATIONAL SEARCH REPORT

International application No.
PCT/JP2005/011094

**Box No. II Observations where certain claims were found unsearchable (Continuation of item 2 of first sheet)**

This international search report has not been established in respect of certain claims under Article 17(2)(a) for the following reasons:

1. [X] Claims Nos.: 1, 3, 5
because they relate to subject matter not required to be searched by this Authority, namely:
Claims 1, 3 and 5 pertain to methods for treatment of the human body by therapy and thus relate to a subject matter which this International Searching Authority is not required, under the provisions of the PCT Rule 39.1(iv), to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**
[ ] The additional search fees were accompanied by the applicant's protest.
[ ] No protest accompanied the payment of additional search fees.

Form PCT/ISA/210 (continuation of first sheet (2)) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004048332 A
- JP 2003 A
- JP 346937 A


### Non-patent literature cited in the description


- **R. PAUSE.** *N. Engl. J. Med.,* 1999, vol. 341, 491-497
- **K.S. STENN et al.** *Physiol. Rev.,* 2001, vol. 81, 449-494
- **S.E. MILLER et al.** *J. Invest. Dermatol.,* 2002, vol. 118, 216-225
- **J. KISHIMOTO et al.** *Proc. Natl. Acad. Sci.,* 1999, vol. 96, 7336-7341
- **EHAMA et al.** *26th Annual Meeting of the Molecular Biology Society of Japan, Abstracts, 2PC-024,* 2003
- **A. TOMAS et al.** *J. Biol. Chem.,* 2003, vol. 278, 20210-21216
- **E.C. BREEN et al.** *J. Cell Biochem.,* 2003, vol. 68, 848-854
- **A. LEASK et al.** *J. Biol. Chem.,* 2003, vol. 278, 13008-13015
- **R. KODYM et al.** *J. Biol. Chem.,* 1999, vol. 274, 5131-5137
- **S. GORUPPI et al.** *Mol. Cell. Biol.,* 2001, vol. 21, 902-915
- **K. NAGAI et al.** *J. Biol. Chem.,* 2003, vol. 278, 18229-18234
- **S.S. BANERJEE et al.** *J. Biol. Chem.,* 2003, vol. 278, 2581-2584
- **J. KACZYNSKI et al.** *Genome Biology,* 2003, vol. 4, 206.1-206.8
- **K. YANO.** *J. Invest. Dermatol.,* 2003, vol. 120, 14-9
- **E.M. CONWAY et al.** *J. Exp. Med.,* 2002, vol. 196, 565-577
- **J.W. FERGUSON et al.** *Mech. Dev.,* 2003, vol. 120, 851-864
- **R.G. LEBARON et al.** *J. Invest. Dermatol.,* 1995, vol. 104, 844-849
- **G. DEL SAL et al.** *Cell,* 1992, vol. 70, 595-607
- **Y. LIU et al.** *Development,* 2002, vol. 129, 5289-5300
- **K.K. LEE et al.** *Dev. Biol.,* 2001, vol. 234, 188-203
- **N. LOPES et al.** *Mol. Cell. Biol.,* 2003, vol. 23, 5401-5408
- **H. XIN et al.** *Oncogene,* 2003, vol. 22, 4775-4785
- **C. LIU et al.** *Mol. Cell. Biol.,* 2000, vol. 20, 7024-7036
- **V.A. BOTCHKAREV.** *J. Invest. Dermatol.,* 2003, vol. 120, 36-47
- **C. DEROANNE et al.** *J. Cell sci.,* vol. 116, 1367-1376
- **N. CARTER et al.** *Nat. Cell Biol.,* 2002, vol. 4, 565-573
- **K.K. MURAI et al.** *Neurosci.,* 2003, vol. 6, 153-160
- **Z. ZHOU et al.** *Genet.,* 2003, vol. 35, 49-56
- **N. INOHARA et al.** *EMBO J.,* 1998, vol. 17, 2526-2533
- **M. LENER et al.** *Eur. J. Biochem.,* 1998, vol. 254, 117-122
- **F.W. INOHARA et al.** *Genomics,* 1993, vol. 15, 507-514
- **T.A. KOROLENKO et al.** *Bull. Exp. Biol. Med.,* 2003, vol. 136, 46-48
- **S. KONDO et al.** *Nat. Gent.,* 2002, vol. 32, 285-289
- **S. CHAKRAVARTI.** *Glycoconj. J.,* 2003, vol. 19, 287-293
- **C. SOO et al.** *Am. J. Pathol.,* 2000, vol. 157, 423-433
- **R. AIZMAN et al.** *Am. J. Pathol.,* 2002, vol. 283, F569-F577
- *Nikkei Science,* April 1944, 20-45
- *Experimental Medicine,* 1994, vol. 12 (15
- Basic Technology of Gene Therapy. Experimental Medicine. Yodosha Publishing, 1996
- *Nikkei Science,* April 1994, 20-45
- *Pharmacology Monthly,* 1994, vol. 36 (1), 23-48
- **KISHIMOTO et al.** *Proc. Natl. Acad. Sci. USA,* 1999, vol. 96, 7336-7341

SEQUENCE LISTING

<110> SHISEIDO CO. LTD.

<120> Method for Regenerating Hair Follicle by Suppressing a Gene with Hair Follicle Formation-Inhibiting Ability or by Activating a Gene with Hair Follicle Formation-Inducing Ability

<130> R748

<150> JP 2004-175407
<151> 2004-06-14

<150> JP 2004-175444
<151> 2004-06-14

<160> 10

<210> 1
<211> 20
<212> DNA
<213> Artificial Sequence
<223> Forward Primer

<400> 1
gagtcaacgg atttggtcgt 20

<210> 2
<211> 20
<212> DNA
<213> Artificial Sequence
<223> Reverse Primer

<400> 2

tgggatttcc attgatgaca 20

<210> 3
<211> 20
<212> DNA
<213> Artificial Sequence
<223> Reverse Primer

<400> 3
gaattctttg acttcactta 20

<210> 4
<211> 20
<212> DNA
<213> Artificial Sequence
<223> Forward Primer

<400> 4
atttgtagag tttgataggt 20

<210> 5
<211> 21
<212> DNA
<213> Artificial Sequence
<223> Forward Primer

<400> 5
tctgggcagt attgctccta c 21

<210> 6
<211> 21
<212> DNA
<213> Artificial Sequence

<223> Reverse Primer

<400> 6
cttgtgggtg tagtgggaga g 21

<210> 7
<211> 21
<212> DNA
<213> Artificial Sequence
<223> Forward Primer

<400> 7
tatttgtccg cactacaaca g 21

<210> 8
<211> 21
<212> DNA
<213> Artificial Sequence
<223> Reverse Primer

<400> 8
aatttttcgg agaacttgat g 21

<210> 9
<211> 20
<212> DNA
<213> Artificial Sequence
<223> Forward Primer

<400> 9
ggggtctttc aagttccaat 20

<210> 10
<211> 20
<212> DNA
<213> Artificial Sequence
<223> Reverse Primer

<400> 10
tcggtaaggg gaacttttct 20